# EUROPEAN PATENT SPECIFICATION

(11) **EP 1 361 216 B1**
(45) Date of publication and mention of the grant of the patent: **16.11.2005**
(21) Application number: 03013275.7
(22) Date of filing: 04.09.1997
(51) Int. Cl.: C07D 217/26

(54) **Method of making a HIV-Protease inhibitor**
Verfahren zur Herstellung von einem HIV-Proteasehemmer
Procede de production d'un inhibiteur de la protease du VIH

(30) Priority: 05.09.1996 US 708411
(43) Date of publication of application: 12.11.2003
(62) Divisional of application: 97941402.6
(73) Proprietor: AGOURON PHARMACEUTICALS, INC., San Diego, California 92121 (US); Japan Tobacco Inc., Takatsuki, Osaka 569 (JP)
(72) Inventor: Deason, Michael E., Poway, CA 92064 (US); Whitten, Kathleen R., San Diego, CA 92130 (US)
(74) Representative: Grünecker, Kinkeldey, Stockmair & Schwanhäusser Anwaltssozietät

(56) References cited:
- EP-A- 0 984 000
- US-A- 5 484 926

## Description

### Introduction

Treatment of HIV-infected individuals is one of the most pressing biomedical problems of recent times. A promising new therapy has emerged as an important method for preventing or inhibiting the rapid proliferation of the virus in human tissue. HIV-protease inhibitors block a key enzymatic pathway in the virus resulting in substantially decreased viral loads, which slows the steady decay of the immune system and its resulting deleterious effects on human health. The HIV-protease inhibitor nelfinavir mesylate of formula 7 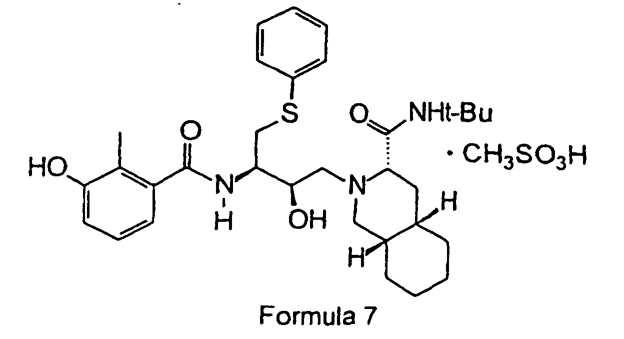 has been shown to be an effective treatment for HIV-infected individuals.

US-A-5,484,926 discloses HIV protease inhibitors, obtainable by chemical synthesis. EP 0 984 000 A1, a document according to Article 54 (3) (4) EPC, discloses a process for producing amide derivatives and intermediate compounds.

### Summary of the Invention

It is an object of this invention to provide an easy method for providing a compound of formula 7.

This object has been solved with the process of claim 1. Preferred embodiments are disclosed in claims 2 to 4 .

The process starts with a compound of formula 4, illustrated below:

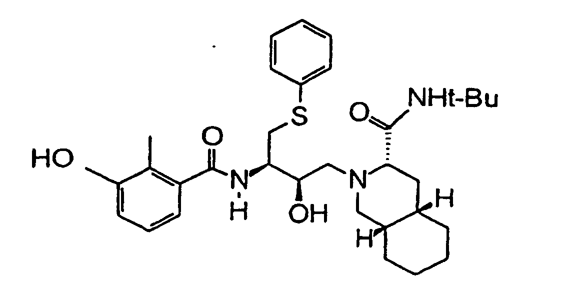

This compound may be prepared starting with a compound of formula 3 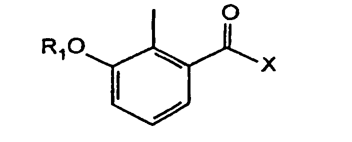 wherein R₁ is alkyl; cycloalkyl; heterocycloalkyl; aryl; heteroaryl; or a group of formula 8 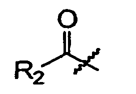 wherein R₂ is an alkyl group, a cycloalkyl group, a heterocycloalkyl group, or O-R₆. wherein R₆ is an alkyl group, an aralkyl group, or an aryl group;
or further wherein R₁ is a group of formula 9 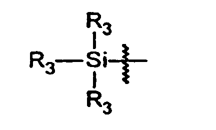 wherein each R₃ independently is an alkyl group, a cycloalkyl group, a heterocycloalkyl group, an aryl group, or a heteroaryl group;
or further wherein R₁ is a group of formula 10 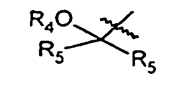 wherein R₄ and each R₅ independently are an alkyl group, a cycloalkyl group, a heterocycloalkyl group, an aryl group, or a heteroaryl group; and
X is OH; OR₇, wherein R₇ is alkyl or aryl; halogen; pseudohalogen; OSO₂ R₈ , wherein R₈ is alkyl or aryl; heteroaryl bonded through the heteroatom; or N-hydroxyheterocyclic bonded through the oxygen,
which is reacted under suitable and sufficient conditions to form the compound of formula 4. For one preferred embodiment of this process, the variable R₁ represents -C(O)CH₃ and/or the variable X represents Cl.

The compound according to formula 4, identified above, also can be prepared by deprotecting a compound of formula 5 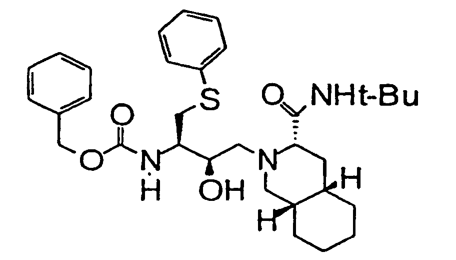 and reacting with it, under sufficient conditions, a compound of formula 3. In this instance, the compound according to formula 3 is 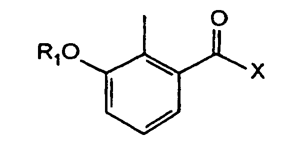 wherein R₁ is alkyl; cycloalkyl; heterocycloalkyl; aryl; heteroaryl; or a group of formula 8 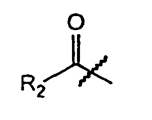 wherein R₂ is an alkyl group, a cycloalkyl group, a heterocycloalkyl group, or O-R₆, wherein R₆ is an alkyl group, an aralkyl group, or an aryl group;
or further wherein R₁ is a group of formula 9 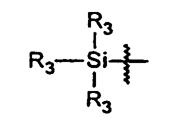 wherein each R₃ independently is an alkyl group, a cycloalkyl group, a heterocycloalkyl group, an aryl group, or a heteroaryl group;
or further wherein R₁ is a group of formula 10 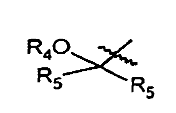 wherein R₄ and each R₅ independently are an alkyl group, a cycloalkyl group, a heterocycloalkyl group, an aryl group, or a heteroaryl group; and
X is OH; OR₇, wherein R₇ is alkyl or aryl; halogen; pseudohalogen; OSO₂R₈, wherein R₈ is alkyl or aryl; heteroaryl bonded through the heteroatom; or N-hydroxyheterocyclic bonded through the oxygen.

In another embodiment of this invention, a compound of formula 4, as identified above, can be prepared by combining a compound of formula 3: 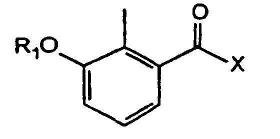 wherein R₁ is alkyl; cycloalkyl; heterocycloalkyl; aryl; heteroaryl; or a group of formula 8 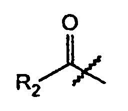 wherein R₂ is an alkyl group, a cycloalkyl group, a heterocycloalkyl group, or O-R₆, wherein R₆ is an alkyl group, an aralkyl group, or an aryl group;
or further wherein R₁ is a group of formula 9 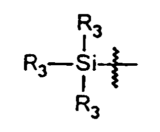 wherein each R₃ independently is an alkyl group, a cycloalkyl group, a heterocycloalkyl group, an aryl group, or a heteroaryl group;
or further wherein R₁ is a group of formula 10 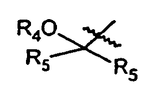 wherein R₄ and each R₅ independently are an alkyl group, a cycloalkyl group, a heterocycloalkyl group, an aryl group, or a heteroaryl group; and
X is OH; OR₇, wherein R₇ is alkyl or aryl; halogen; pseudohalogen; OSO₂R₈, wherein R₈ is alkyl or aryl; heteroaryl bonded through the heteroatom; or N-hydroxyheterocyclic bonded through the oxygen,
with a compound of formula 6 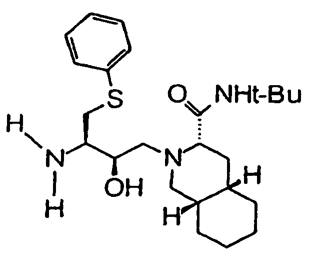 under conditions sufficient and suitable to obtain the compound of formula 4.

This invention relates to a method of making a compound of formula 7. In one embodiment, the compound of formula 7 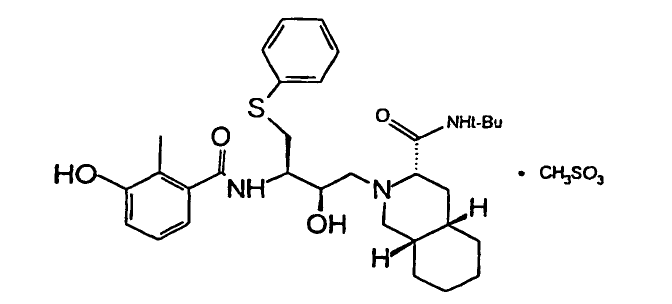 is produced by converting a compound of formula 4 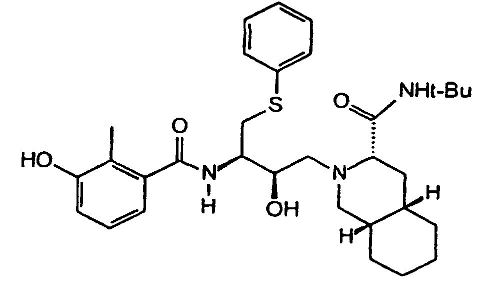 under sufficient and suitable conditions to the compound of formula 7. In this method, the conversion of the compound of formula 4 to the compound of formula 7 takes place by:
(a) contacting the compound of formula 4 with an organic solvent;
(b) contacting the compound of formula 4 with methanesulfonic acid under conditions sufficient to form a compound of formula 7; and
the compound of formula 7 being dissolved in solution;
(b) a first antisolvent is added to the solution containing the compound of formula 7;
(c) the compound of formula 7 and the first antisolvent are agitated together to form a product having a solid phase and a liquid phase; and
(d) the product is filtered and washed with a second antisolvent, the second antisolvent being the same as or different from the first antisolvent, to obtain a solid final product according to formula 7. After the solid final product is washed, it can be dried by any appropriate method or means. Tetrahydrofuran can be used as the solvent, and diethylether can be used as at least one antisotvent, preferably at least the first antisolvent.

### Preparation of Nelfinavir Free Base from 3-Acetoxy-2-Methylbenzoic Chloride

### Summary of the Process

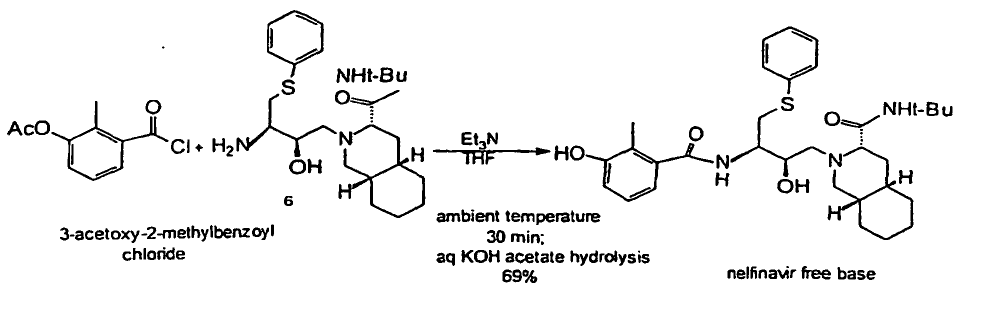

To obtain 3-acetoxy-2-methytbenzoyl chloride, 3-hydroxy-2-methylbenzoic acid was slurried in acetic acid with acetic anhydride and catalytic sulfuric acid. Acetylation of the hydroxy group was complete within two hours at ambient temperature. After complete reaction, the resulting slurry was poured into water, and the product was isolated by filtration. The wet cake was reslurried in water, isolated by filtration, and dried under vacuum. Product was obtained in 80-90 % yield with an apparent purity of 89-92% by HPLC. Crude, dry 3-acetoxy-2-methylbenzoic acid was dissolved in four volumes of ethyl acetate with refluxing. The resulting solution was cooled to <70 °C, and five volumes of hexanes were added. The mixture was returned to reflux and then cooled to <10 °C for 1 hour. The slurry was filtered, rinsing the reactor with filtrate. The product was dried under vacuum. Recrystallization improved the HPLC UV apparent purity from 89 - 92% to >98%. The single largest impurity dropped from 4 - 5% to -0.5%. The product was 3-acetoxy-2-methylbenzoic acid.

3-Acetoxy-2-methylbenzoic acid was slurried in methyl-t-butyl ether (MTBE) and treated with 1.2 equivalents of thionyl chloride and catalytic dimethylformamide. After three hours at ambient temperatures, the reaction was complete, giving a brown solution. Solvent (MTBE) was removed by vacuum distillation. Residual thionyl chloride was removed by addition of toluene followed by vacuum distillation. The resulting 3-acetoxy-2-methylbenzoyl chloride was isolated either directly as an oil or by crystallization from two volumes of heptane at <10 °C. Product was obtained in >100% yield when isolated as an oil and 82 - 85% yield when crystallized from heptane.

To obtain compound 6 for the coupling, a compound of formula 5 (made as described below) was refluxed in a mixture of ethanol and aqueous NaOH to cleave the CBZ protecting group forming a compound of formula 6. Water and HCl were added to dissolve the Na₂CO₃ and neutralize excess NaOH, giving a biphasic mixture. The mixture was cooled, and the lower aqueous layer was removed. Triethylamine was added followed by a solution of 3-acetoxy-2-methylbenzoyl chloride in tetrahydrofuran to give an acetate of the compound of formula 4. Aqueous NaOH was added, and the mixture was heated to reflux to give a compound of formula 4. The mixture was concentrated at atmospheric pressure to remove tetrahydrofuran, triethylamine, and most of the ethanol. The mixture was added to a heated solution of water and glacial acetic acid to precipitate the product. The pH was adjusted with additional acid, and the solids were filtered off while hot. The wet cake was rinsed with hot water and dried to give crude nelfinavir free base.

This method is described in more detail below

### Preparation of 3-Acatoxy-2-methylbenzoic acid

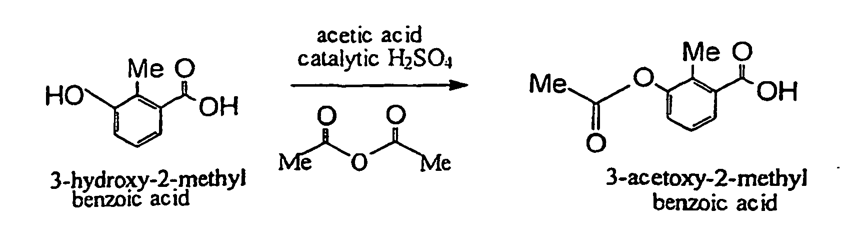

### PROCEDURE:

### Materials

| | | | | |
|---|---|---|---|---|
| 3-hydroxy-2-methylbenzoic acid | FW 152.15 | | 3500 g | 1.0 equiv |
| acetic acid | | | 8750 mL | |
| sulfuric acid | | | 70 mL | |
| acetic anhydride | FW 102.1 | d 1.082 | 2390 mL | 1.1 equiv |
| purified water | | | 28000 mL | |

Acetic acid (8750 mL), 3-hydroxy-2-methylbenzoic acid (3500 g), and sulfuric acid (70 mL) were charged into a 22 liter reactor. The reactor contents were stirred to give a homogeneous mixture. The mixture exothermed to 36 °C. Acetic anhydride (2390 mL) was added to the mixture in the 22 L reactor. An exotherm warmed the reactor contents from 36 to 44 °C. The reaction mixture was stirred at ambient temperature for two hours (reactor contents allowed to cool slowly). The reaction was tested for complete conversion of the starting material by TLC. The reaction mixture was generally a tan slurry at the completion of the reaction.

Purified water (17500 mL) was added to a 50 L extractor, and the reaction mixture from the 22 L reactor was added to this water. The 22 L reactor was rinsed into the 50 L extractor with purified water (3500 mL). The reaction mixture was vacuum filtered, washing the reactor and filter cake with purified water (3500 mL). The wet filter cake was transferred to a 50 L extractor, and purified water (14000 mL) was added, with stirring, to obtain a homogeneous slurry. The reslurried mixture was vacuum filtered, and the reactor and filter cake were rinsed with purified water (3500 mL). The filter cake was pulled as dry as possible and then transferred to drying pans. The product was dried in a vacuum oven at 60 - 80 °C and ≥28 mm Hg for 12 - 72 hours. Theoretical yield: 4466 g. Actual weight produced: 3910 g (87.6%). HPLC assay: 89.4% or 87.7%.

Purification was achieved as follows. The crude 3-acetoxy-2-methylbenzoic acid (3910 g from above) and ethyl acetate (16.0 L) were charged to a 50 L reactor. The reactor contents were heated to reflux (77 °C) until all solids went into solution. The reactor contents were cooled to < 70 °C. Hexanes (19.5 L) were added to the reactor. The reactor contents were again heated to reflux (69 °C), and then the mixture was cooled to <10 °C for 1 hour. The cooled slurry from this step was vacuum filtered, and the reactor was rinsed with cold mother liquors. The filter cake was pulled as dry as possible and then transferred to drying pans. The product was dried in a vacuum oven at 60 - 70 °C and ≥28 mm Hg for 12 - 72 hours. Theoretical yield: 3910 g. Actual weight produced: 3128 g (80%). This procedure improves the HPLC UV apparent purity from 89 - 92% to >98%. The single largest impurity drops from 4 - 6% to <1%. The isolated product is a tan solid. ¹H NMR δ 8.0 (d, 1H), 7.3 (overlapping m, 2H), 2.5 (s, 3H), 2.3 (s, 3H).

### Preparation of 3-Acetoxy-2-methylbenzoyl Chloride

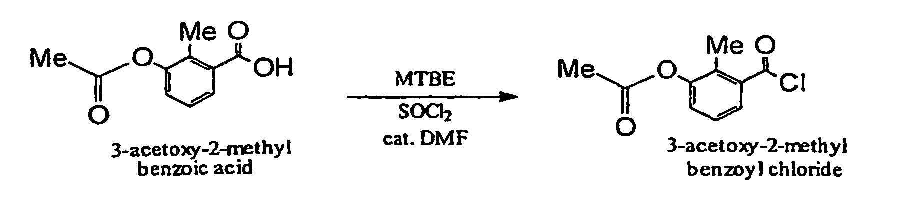

### PROCEDURE:

| Materials | MW | d | wt | equiv. |
|---|---|---|---|---|
| 3-acetoxy-2-methylbenzoic acid | 194.19 | | 3000 g | 1.0 |
| methyl-*t*-butyl ether | | | 12000 ml | |
| thionyl chloride | 118.97 | 1.638 | 1350ml | 1.2 |
| dimethylformamide | 73.09 | 0.944 | 60 ml | 0.05 |
| toluene | | | 7500 ml | |
| heptane | | | 7500 ml | |

A 22 L reactor was purged with nitrogen and charged with recrystallized 3-acetoxy-2-methylbenzoic acid (3000 g), MTBE (12000 ml), and dimethylformamide (60 ml). The reactor contents were stirred to give a homogeneous mixture. Thionyl chloride (1350 ml) was added to the reactor. This reaction mixture was stirred at ambient temperature for 19 hours. (Generally no more than 3 hours are required for complete reaction, but the mixture can be held longer for convenience). The reaction solution was transferred to a Büchi rotovap, and the reactor was rinsed with toluene (1500 ml). The solution was concentrated as far as possible, maintaining the bath temperature at 40 - 50 °C. Toluene (6000 ml) was added to this concentrated solution. The toluene was distilled by rotovap to drive off excess thionyl chloride. The concentrate was transferred back to the 22 L reactor, and the Büchi flask was rinsed with heptane (6000 ml). The heptane mixture was cooled to <5 °C under nitrogen. After holding the crystallization mixture at <5 °C for > 30 minutes, the mixture was filtered, and the filter cake was washed with chilled heptane (1500 ml, <5 °C). The filter cake was dried in a vacuum oven at 15 - 20 °C and ≥28 mm Hg for 24 hours, giving a tan, granular solid. Theoretical yield: 3285 g. Actual weight produced: 2704 g (82.3%). HPLC assay 97.51 %; ¹H NMR δ 8.1 (d, 1H), 7.4 (overlapping m, 2H), 2.4 (s, 6H).

### Conversion of 3-Acetoxy-2-methylbenzoyl Chloride and Compound-6 to Nelfinavir Free Base

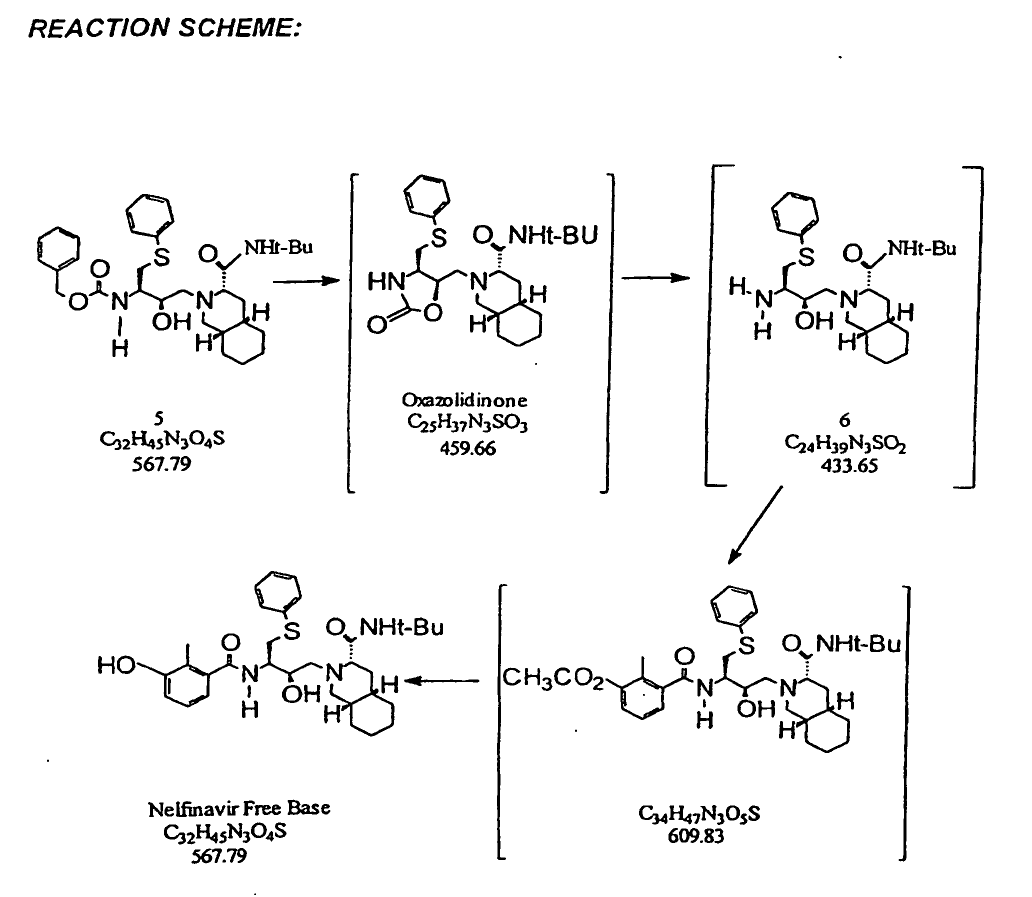

### Step A: Conversion of Compound 5 to Compound 6.

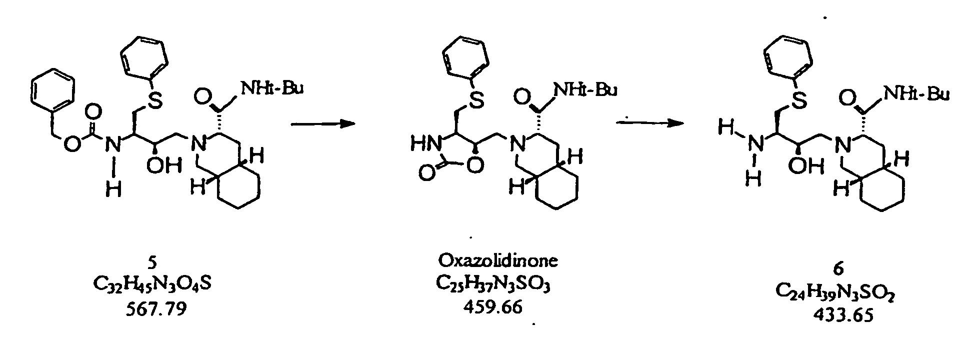

A 22 liter 3-neck round bottom flask ("RBF"), fitted with a condenser, temperature probe, and mechanical stirrer, was charged with a compound of formula 5 (1 kg, 97.7%, 1.72 mol. 1.00 eq) (which can be prepared as described below), ethanol (5 l, 95%), NaOH (280 ml, 50%, 5.33 mol, 3.1 eq), and water (2 l, DI). The mixture was stirred and heated to reflux (80-82 °C). All solids dissolved at 50 °C to give a clear yellow solution. The mixture became hazy with Na₂CO₃ precipitation as the reaction proceeded (Vol = 8280 ml). The deprotection was monitored by HPLC. Analysis at 210 minutes showed complete consumption of the compound of formula 5, 0.95% oxazolidinone, 36% benzyl alcohol, and 62.5% of a compound of formula 6. Analysis at 300 minutes showed 0.34% oxazolidinone, 36% benzyl alcohol and 62.6% of compound of formula 6. Water (1260 ml) was added to the mixture to dissolve all solids, and the mixture was cooled to 67 °C (Vol = 9540 ml). HCl (344 ml, 6 N, 2.06 mol, 1.2 eq.) was then added to the mixture. The mixture was agitated 10 minutes and then allowed to settle for 20 minutes to give two layers (Vol = 9884 ml). The lower aqueous Na₂CO₃ layer was removed at 60 °C. The volume of the aqueous cut was 365 ml, pH = 14. The pH of the clear yellow upper layer was 10-10.5. The upper layer was used directly in the next step.

### Step B: Conversion of Compound 6 to an Acetate of Compound 4.

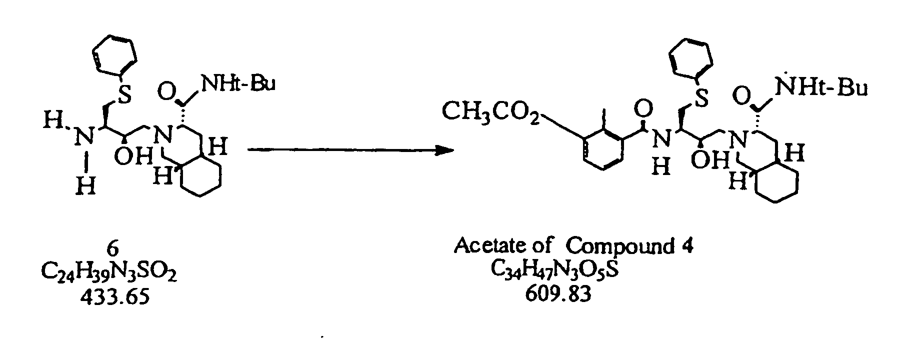

| Chemicals | | | | | | | | |
|---|---|---|---|---|---|---|---|---|
| | source | assay | Kg | L | d | mw | mol | equiv |
| Compound 6 /EtOH | 1040-090 | | (0.746) | | | 433.65 | (1.72) | 1.00 |
| triethylamine | Fisher | | 0.26 | 0.36 | 0.726 | 101.19 | 2.58 | 1.50 |
| tetrahydrofuran | Fisher | | 0.40 | 0.45 | 0.889 | 72.11 | | |
| 3-acetoxy-2-methylbenzoyl chloride | AC1322 | 98.9% | 0.39 | | | 212.63 | 1.81 | 1.05 |
| Reference: | 1040-092 | | | | | | | |

The solution from Step A was cooled to 25 °C, triethylamine (360 ml, 2.58 mol, 1.50 eq) was added to the solution, and the mixture was cooled to 7 °C (pH = 11.5-12.0). The mixture became hazy at 23 °C (Vol = 9879 ml). This mixture was charged to a mixture of 3-acetoxy-2-methylbenzoyl chloride (388.5 g, 98.8 %, 1.81 mol, 1.05 eq) and tetrahydrofuran (440 ml) over 5 minutes. THF (10 ml) was used to complete the transfer. A 7.4 °C exotherm was observed. The mixture at the end of the addition was milky white. (Vol = 10,717 ml). HPLC analysis after 30 minutes showed <0.2% of a compound of formula 6, 77% of the acetate of the compound of formula 4,18.2% benzylalcohol, and no ester present. The milky mixture was used directly in the next step.

### Step C : Saponification of Compound 4.

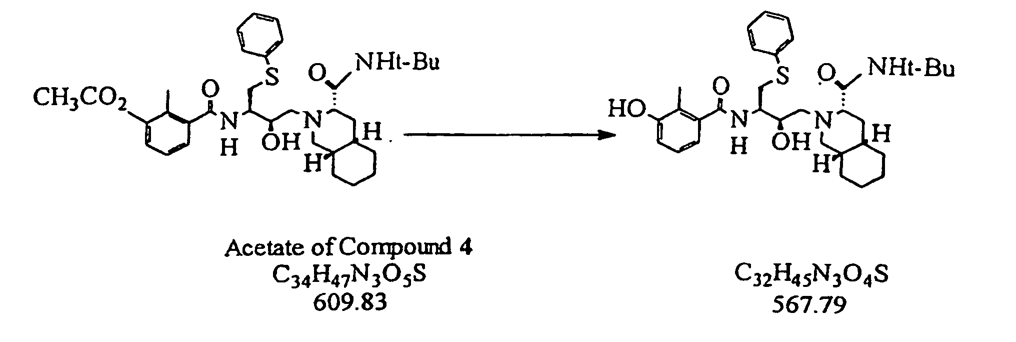

| **Chemicals** | | | | | | | | |
|---|---|---|---|---|---|---|---|---|
| | **source** | **assay** | **Kg** | **L** | **d** | **mw** | **mol** | **equiv** |
| Acetate of | | | | | | | | |
| Compound 4/PA | 1040-092 | | (1.05) | | | 609.83 | (1.72) | 1.00 |
| NaOH | Fisher | 50% | 0.55 | 0.36 | 1.515 | 40.00 | 6.88 | 4.00 |
| Water | DI | | 15.0 | 15 | 1.000 | 18.02 | | |
| HOAc, glacial | Fisher | 17.4 N | 0.25 | 0.23 | 1.049 | 60.05 | 4.07 | 2.37 |
| Ethanol. (5% methanol) | McCormick | 95% | 0.04 | 0.05 | 0.785 | 46.07 | | |

NaOH (50%, 364 ml, 6.88 mol, 4.0 eq) was added to the mixture from Step B. The milky mixture became clear, then hazy, light brown. The mixture was agitated at 20°C for 35 minutes. HPLC showed 15.9% benzylalcohol, 78.6% of compound 4, and no acetate (Vol = 11,081 ml). The mixture was heated to reflux and partially concentrated by atmospheric distillation until the head temperature reached 82°C. The distillate volume was 4275 ml. The pH of the mixture was 14. The pot volume was measured (Vol = 6000 ml).

Water (5 L) and HOAc (100 mL) were charged to a 12 L 3-neck round bottom flask fitted with a temperature probe and mechanical stirrer. The solution was heated to 54 °C (pH = 2-2.5) (Vol = 5100 ml). One half of the compound 4 mixture produced above (3 L) was added to this warm aqueous acetic acid solution to precipitate fine white solids. The pH was then adjusted to 7-7.5 with HOAc (19 ml), and the temperature was 53 °C (Vol = 8119 ml). The solids were filtered off at 53 °C using isolated vacuum. The filtration was quick and easy. The reactor and wet cake were rinsed with warm (35 °C) water (2.5 L), and the filtrates were combined. The wet cake was pulled dry for 15-20 minutes.

Water (5 L) and HOAc (100 mL) were charged again to the 12 L 3- neck round bottom flask. The solution was heated to 41 °C (Vol = 5100 ml). The remaining half of the compound 4 reaction mixture (3 L) was added to the new warm aqueous acetic acid solution to precipitate fine white solids. The pH was then adjusted to 7-7.5 with HOAc (15 ml). The temperature was 44 °C (Vol = 8115 ml). The solids were filtered off at 53 °C using isolated vacuum. The filtration was quick and easy. The reactor and wet cake were rinsed with warm (35 °C) water (2.5 L), and the filtrates were combined. The wet cake was pulled dry for 15-20 minutes.

The two wet cakes (3587 g) were dried under vacuum at 60 °C for 90 hours to give a dry wt of 1075.38 g crude Compound 4. Theoretical yield is 977 g.

### Step D : Purification of Compound 4

| **Chemicals** | | | | | | | | |
|---|---|---|---|---|---|---|---|---|
| | **source** | **assay** | **w t** | **ml** | **d** | **mw** | **mmol** | **equiv** |
| Crude Compound 4 | 895-131 | 91.82% | 290 g | | | 567.79 | 469 | 1.00 |
| Acetone | Fisher | | 4038 g | 5105 | 0.791 | 58.08 | | |
| Water | DI | | 1070 g | 1070 | 1.000 | 18.02 | | |
| Celite | Aldrich | | 29 g | | | | | |
| Darco G-60 activated carbon | Fisher | | 44 g | | | 12.01 | | |

A 12 liter 3-neck RBF, fitted with a condenser, temperature probe, and mechanical stirrer, was charged with crude compound 4 (290 g, 92%, 469 mmol), activated carbon (Darco G-60, 44g), acetone (4305 ml), and water (870 ml, DI). The mixture was heated to reflux (60-64°C) and held 45 minutes (Vol = 5509 ml). The hot slurry was filtered through celite (29 g) using isolated vacuum. The reactor and filter cake were rinsed with acetone (200 ml), and the clear, light yellow filtrates were combined. The mixture was allowed to cool slowly to 25°C over 2.5 hours with stirring to precipitate a fine white solid (Vol = 5665 ml). The white slurry was cooled to 0-10 °C and held for 1 hour. The solids were filtered off using isolated vacuum, and the liquid level was pulled through the surface of the wet cake. The reactor and wet cake were rinsed with a cold (0- 1.0 °C) mixture of acetone/water (2:1, 300 ml). The liquid level was pulled through the surface of the wet cake, and the reactor and wet cake were again rinsed with a cold (0-10 ° C) mixture of acetone/water (2:1, 300 ml). The wet cake was pulled as dry as possible using isolated vacuum and rubber damming to give a wet weight of 581 g. The product was dried under vacuum at 65 °C for 16 hours to give a dry weight of 221.61 g of compound 4. Theoretical yield was 266.28 g. HPLC and ROI analysis showed 99% and 0.14% respectively. Adjusted yield was 82%.

The present invention also is directed to novel methods of converting nelfinavir free base, compound 4, to nelfinavir mesylate, compound 7. These methods are described in more detail below, including the method for preparing compound 4 from compound 5 and the method for preparing compound 5.

### Procedure for Preparation of Compound 5

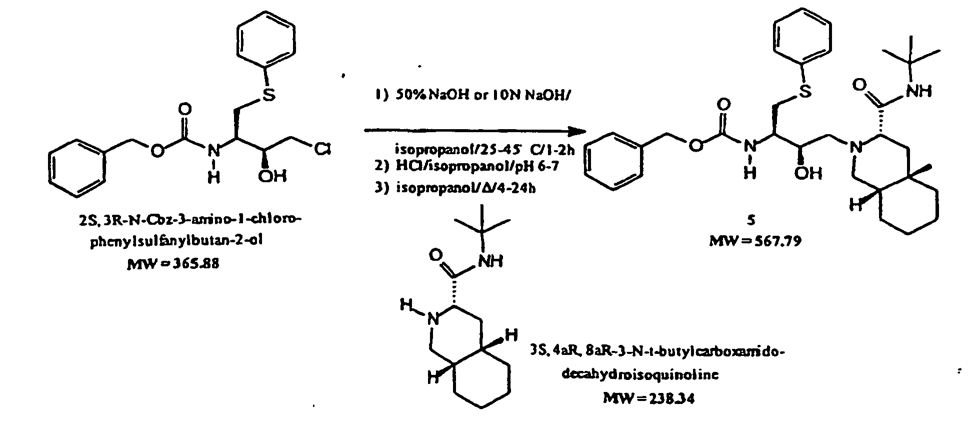

One equivalent 2S, 3R-N-Cbz-3-amino-1-chlorophenyl-sulfanylbutan-2-ol (which can be obtained from Kaneka Corporation or prepared as described in U.S. Patent No. 5,484,926) is stirred in a sufficient volume of methanol, ethanol, isopropanol, or other low boiling alcoholic solvent at 20° - 45°C. Isopropanol is the preferred solvent. A slight subcess of alkali base, such as sodium hydroxide or potassium hydroxide, as either an aqueous solution or as a solid, is added to this mixture with stirring. 10N sodium hydroxide is the preferred base. The mixture is stirred for 30 minutes to 24 hours until epoxide formation is complete. When the stir period is complete, the pH is adjusted to 6- 7 with a proton acid such as HCl, either neat or dissolved in the reaction solvent.

A slight excess of 3S,4aR,8aR-3-N-t-butylcarboxamidodecahydroisoquinoline (which can be prepared as described in U.S. Patent No. 5,256,783, which patent is entirely incorporated herein by reference) is added as either a dry solid or as a slurry to the reaction, and the mixture is heated to 40°C to reflux for 12-24 hours or until the reaction is judged to be complete. Altematively, 3S,4aR,8aR-3-N-t-butylcarboxamidodecahydroisoquinoline can be introduced to the reaction at the same time that the 2S, 3R-N-Cbz-3-arnino-1-chlorophenylsulfanylbutan-2-ol is charged to reactor. The epoxide formation is allowed to proceed as described. In this case, the reaction is not neutralized to a pH of 6-7, but a fixed amount of proton acid is added to neutralize excess base remaining. In either case, the reaction is partially concentrated *in vacuo.* The mixture is diluted with an equal volume of water and heated to reflux. Alternatively, the reaction is fully concentrated, and acetone or other ketonic solvent is added. The mixture can be filtered at this point, then an equal amount of water is added, and the mixture is heated. The resultant mixture is cooled with stirring. The resultant slurry is filtered, washed with aqueous solvent, and dried to yield compound 5.

### Procedure for Preparation of Nelfinavir Free Base (Compound 4)

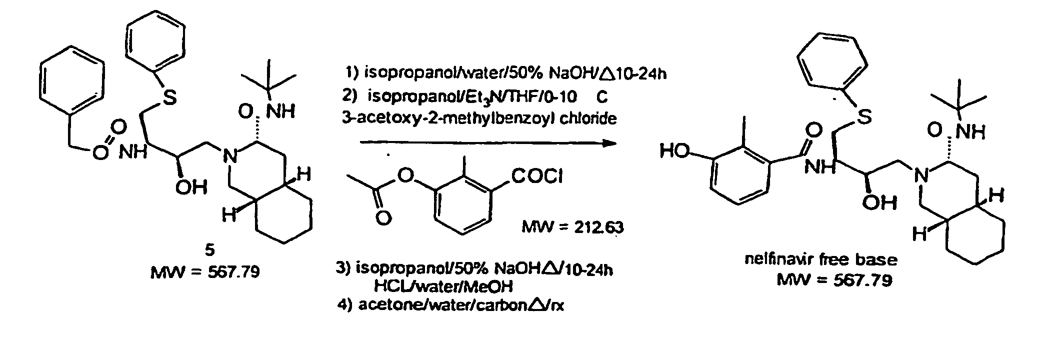

In addition to the procedure described above, the following procedure can be used to convert compound 5 to the nelfinavir free base (compound 4):

One equivalent of compound 5, an excess of alkali base (such as sodium hydroxide or potassium hydroxide), and an alcoholic solvent (such as methanol, ethanol, or isopropanol) are combined, and the mixture is heated at reflux with stirring. 50% caustic soda is the preferred base and isopropanol is the preferred solvent. Water can be added to facilitate solubility of the base. When the reaction is judged complete, the mixture is cooled to 30° to 35°C, and the lower aqueous layer, if any, can be removed. The mixture is cooled to below 25°C and an excess amount of organic base (such as diisopropylethyl amine or triethylamine) is added. Triethylamine is the base of choice.

A solution of excess 3-acetoxy-2-methylbenzoyl chloride in methanol, ethanol, isopropanol, THF, or other alcohol soluble solvents is slowly added to the cold mixture with stirring. THF is the preferred solvent.

An excess of alkali base, such as sodium hydroxide or potassium hydroxide, is added, and the mixture is heated at 40°C to reflux with stirring. 50% caustic soda is the preferred base. When the reaction is judged complete, the mixture is cooled, and the lower aqueous layer is removed.

The reaction mixture is partially concentrated *in vacuo.* If deemed necessary, the mixture can be diluted with an alcohol solvent to facilitate stirring. Methanol is the preferred solvent. The mixture is added to aqueous acid to form a slurry. HCl is the preferred acid. The pH is adjusted to 7-8 with aqueous acid. The slurry is filtered and washed with water. The wet cake can be reslurried in water. The crude product is dried (partially or completely) or can be taken into the next step wet.

Either the dry or the crude, wet product is dissolved in aqueous acetone at reflux in the presence of activated carbon. The hot mixture is filtered, water is added, and the entire mixture is cooled with stirring to form a slurry. The slurry is filtered, washed with aqueous acetone, and dried to give nelfinavir free base.

### Procedure for Precipitation of Nelfinavir Free Base to Obtain Nelfinavir Mesylate

Nelfinavir free base can be converted to nelfinavir mesylate using the following novel precipitation procedure.

Nelfinavir free base is slurried or dissolved in a suitable solvent (such as THF, methanol, or ethanol). THF is the preferred solvent. A molar equivalent amount of methanesulfonic acid is added, and the mixture is stirred until all solids dissolve. The solution is added to several volumes of an antisolvent selected from diethyl ether, hexanes, or heptanes, that is rapidly stirring. Diethyl ether is the preferred antisolvent. After stirring, the mixture is filtered and washed with antisolvent. The solid is dried in a vacuum oven to yield nelfinavir mesylate:

Specifically, this conversion was performed as described below.

Nelfinavir free base (10.2 kg, 18.0 mol) and 24 L of tetrahydrofuran were added to a 100L reactor. Methanesulfonic acid (1.8 kg, 18.48 mol) also was added to the reactor. The reactor was stirred until all solids dissolved, and then the solution was filtered into a 100 gallon polypropylene tank containing 306 L diethyl ether that was rapidly stirring. After stirring for 2 hours, the 100 gallon tank contents were filtered, washed with 17 L of diethyl ether, and pulled as dry as possible. The solid was transferred to a rotocone drier and dried in a vacuum oven at 60-65°C (at least 26 in. Hg or higher vacuum) for 12-72 hours or until the diethyl ether content of the dried solid was below 1%. If necessary, the drier contents could be milled in a Fitzmill grinder to accelerate drying. Typical yields of nelfinavir mesylate range from 9 to 11 kg. (76% - 92% theory).

## Claims

1. A method of making a compound of formula 7 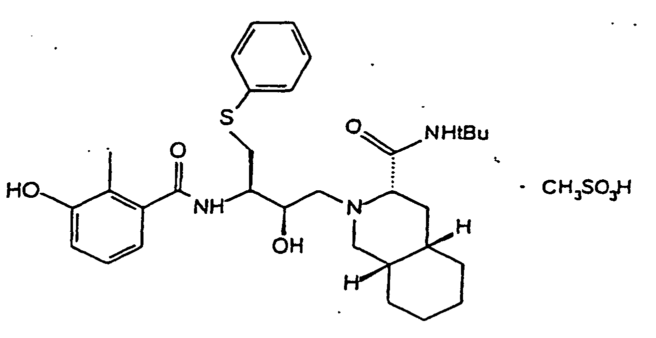 comprising: converting under sufficient conditions a compound of formula 4 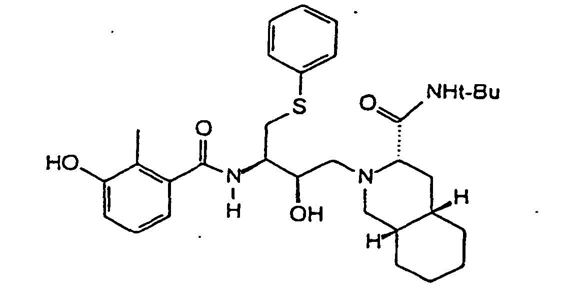 to the compound of formula 7 by:
(a) combining the compound of formula 4, a suitable solvent, and methanesulfonic acid to form the compound of formula 7, the compound of formula 7 being dissolved in solution;
(b) adding a first antisolvent to the solution containing the compound of formula 7, wherein the antisolvent is selected among diethyl ether, hexanes and heptane ;
(c) agitating the compound of formula 7 and the first antisolvent to form a product having a solid phase and a liquid phase; and
(d) filtering and washing the product with a second antisolvent, the second antisolvent being the same as or different from the first antisolvent, to obtain a solid.

2. A method according to claim 1, the method additionally comprising:
(e) drying the solid.

3. A method according to claim 1, wherein the suitable solvent is tetrahydrofuran.

4. A method according to claim 1, wherein the first antisolvent is diethyl ether.

## Patentansprüche

1. Verfahren zum Herstellen einer Verbindung der Formel 7 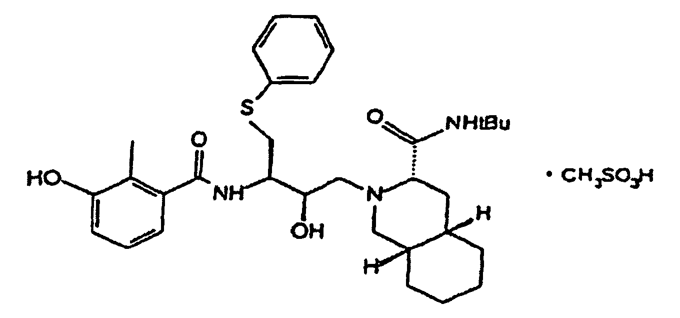 umfassend: das Umwandeln einer Verbindung der Formel 4 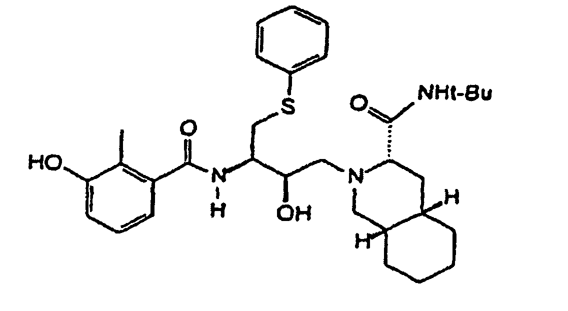 unter hinreichenden Bedingungen in die Verbindung der Formel 7 durch:
(a) das Vereinigen der Verbindung der Formel 4, eines geeigneten Lösungsmittels und von Methansulfonsäure unter Bildung der Verbindung der Formel 7, wobei die Verbindung der Formel 7 in Lösung aufgelöst ist;
(b) das Zugeben eines ersten Antisolvens zu der Lösung, welche die Verbindung der Formel 7 enthält, wobei das Antisolvens ausgewählt ist aus Diethylether, Hexanen und Heptanen;
(c) das Rühren der Verbindung der Formel 7 und des ersten Antisolvens unter Bildung eines Produkts mit einer festen Phase und einer flüssigen Phase; und
(d) das Filtrieren und Waschen des Produkts mit einem zweiten Antisolvens, wobei das zweite Antisolvens das gleiche wie das erste Antisolvens oder davon verschieden ist, um einen Feststoff zu erhalten.

2. Verfahren nach Anspruch 1, wobei das Verfahren zusätzlich umfasst:
(e) das Trocknen des Feststoffs.

3. Verfahren nach Anspruch 1, wobei das geeignete Lösungsmittel Tetrahydrofuran ist.

4. Verfahren nach Anspruch 1, wobei das erste Antisolvens Diethylether ist.

## Revendications

1. Procédé de production d'un composé de formule 7 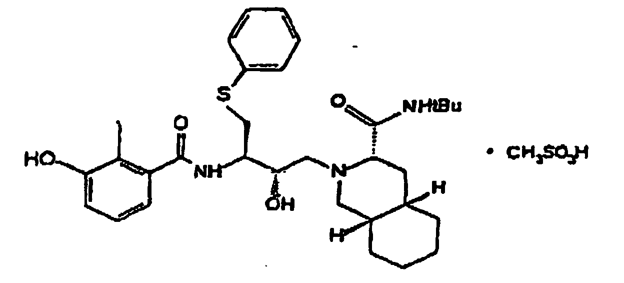 comprenant la transformation, dans des conditions suffisantes, d'un composé de formule 4 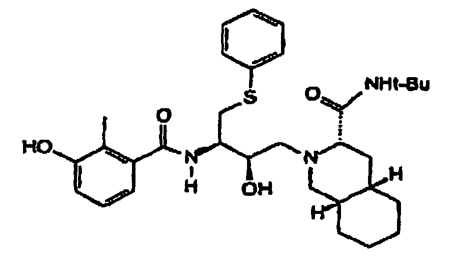 en le composé de formule 7 :
(a) en combinant le composé de formule 4, un solvant adapté et de l'acide méthane sulfonique pour former le composé de formule 7, le composé de formule 7 étant dissous en solution ;
(b) en ajoutant un premier antisolvant à la solution contenant le composé de formule 7, dans lequel l'antisolvant est choisi parmi l'éther diéthylique, les hexanes et les heptanes ;
(c) en agitant le composé de formule 7 et le premier antisolvant pour former un produit ayant une phase solide et une phase liquide ; et
(d) en filtrant et en lavant le produit avec un second antisolvant, le second antisolvant étant identique au premier antisolvant ou différent de celui-ci, pour obtenir une matière solide.

2. Procédé selon la revendication 1, le procédé comprenant de plus :
(e) le séchage de la matière solide.

3. Procédé selon la revendication 1, dans lequel le solvant adapté est le tétrahydrofuranne.

4. Procédé selon la revendication 1, dans lequel le premier antisolvant est l'éther diéthylique.
